Europäisches Patentamt

European Patent Office

Office européen des brevets

(11) Publication number: **0 417 930 A1**

# EUROPEAN PATENT APPLICATION

(21) Application number: 90309377.1

(22) Date of filing: 28.08.90

(51) Int. Cl.⁵: **A61K 9/06**, A61K 37/43

(30) Priority: 29.08.89 JP 222283/89

(43) Date of publication of application:
20.03.91 Bulletin 91/12

(84) Designated Contracting States:
AT BE CH DE DK ES FR GB GR IT LI LU NL SE

(71) Applicant: SUMITOMO PHARMACEUTICALS
COMPANY, LIMITED
2-8, Doshomachi 2-chome, Chuo-ku
Osaka-shi Osaka 541(JP)

(72) Inventor: Fujioka, Keiji
1-27-5-206, Tsukaguchicho
Amagasaki-shi, Hyogo 661(JP)
Inventor: Takada, Yoshihiro
4-37-1-813, Senriyamanishi
Suita-shi, Osaka 565(JP)
Inventor: Aisaka, Ayumi
2-1, Kikusuidori
Moriguchi-shi, Osaka 570(JP)

(74) Representative: Kearney, Kevin David
Nicholas et al
KILBURN & STRODE 30 John Street
London, WC1N 2DD(GB)

(54) Low-irritative nasal preparation.

(57) A GRF-containing nasal preparation is improved in irritation to nasal mocosa by adding sodium chloride and/or sugar alcohols such as mannitol or sorbitol thereto.

EP 0 417 930 A1

## LOW-IRRITATIVE NASAL PREPARATION

This invention relates to a low-irritative nasal preparation of growth hormone releasing factor (hereinbelow referred to as GRF).

GRF is a peptide which shows growth hormone releasing activity in a living body and temporary or long term administration thereof has been tried from a standpoint of clinical medicines of human being and veterinary for diagnosis or treatment.

GRF is so far administered mainly by injections, since it is bioactive peptide and is easily inactivated by proteases and hardly absorbed when it is orally administered. However, a development in a more convenient administration method other than injections is expected, since injection is painful and is inconvenient.

Recently, attention has been paid to intranasal administration so far as peptide and protein drugs are concerned. It is found that GRF is able to be absorbed through nasal mucosa, and various investigations have been made in this respect. However, an application of an aqueous GRF solution obtained by simply dissolving GRF in a water is not useful, since retention time of the solution in the nasal cavity is so short and molecular weight of GRF is so large that absorption rate of GRF is not high. Accordingly, it is necessary for a nasal preparation of GRF to make concentration of GRF in the preparation as high as possible and to apply a large amount of GRF in order to have GRF absorbed across nasal mucous membrane. However, GRF is somewhat irritative to mucosa and a nasal preparation of GRF having enough concentration for clinical effects gives no good feeling after being administered. It is difficult to apply aqueous solution of concentrated GRF for practical use as a nasal preparation as it is, for example, for the treatment of dwarfism, since there is a possibility to injure nasal mucosa due to administration for a long period.

One of approaches is addition of absorption enhancers in order to improve bioavailability of hardly absorbable drugs such as peptides and polypeptides. As to GRF, addition of materials such as saponin or bile acid salts to the preparation are proposed in order to promote absorption rate through nasal mucosa (JP 62-126135). However, these absorption enhancers are, in themselves, somewhat locally toxic and irritative to mucous membranes. Their irritation are rather stronger than those of GRF. Accordingly, the addition of these absorption enhancers is not practical, since nasal mucosa is rather more irritated even if GRF concentration be lowered, and there is a fear of injurious influence on nasal mucosa after a long term repeated administrations. Another approach is to decrease relative osmotic pressure of the preparations to not more than 1, preferably not more than 0.5 in order to improve absorption of GRF, through nasal mucosa into blood circulation (JP 63-303931) where local toxicity and irritation are not so large. However, the latter approach is not always suitable for less irritative preparations, since wider range of osmotic pressure is required from a practical point of view, depending on varieties and desired concentrations of GRF.

There is another problem on GRF solubility in making preparations of GRF, besides irritation. Solubility of GRF is influenced by kinds and concentrations of materials coexisted in aqueous solution, and by ionic strength, pH, etc. Solubility of GRF may decrease according to kinds and concentrations of the materials added. Materials which are active for decreasing irritation of GRF are not suitable as an additive, as long as solubility of GRF is decreased by addition thereof, since no aqueous solution or suspension of GRF having concentration and homogeneniety enough for clinical application is obtained. Almost all preparations to be applied to mucous membranes such as nasal preparations and eye drops are controlled with respect to irritation thereof by addition of various kinds of organic or inorganic salts to such a level that osmotic pressure of the preparations is almost the same as the physiological osmotic pressure. In the case of GRF, however, no desired preparation is obtained depending on inorganic salts which are usually employed as isotonizers, because some of them hinder GRF from being dissolved and GRF is crystalized or precipitated.

It is necessary to consider this sort of property of GRF in the investigation of methods for decreasing irritation of a nasal preparation of GRF. It has been very difficult to develop a nasal preparation of GRF, since properties of materials which are to be used as additives have to be taken into consideration in the case of GRF.

Although a nasal preparation of GRF practically applicable as clinical medicines is expected, such preparation has not yet been in the market because of its irritation to mucosa and difficulty in preparation.

After strenuous effort to obtain a nasal preparation of GRF without irritation to mucosa, the present inventors have unexpectedly found that addition of sodium chloride and/or sugar alcohols serves to decrease irritation of a nasal preparation of GRF.

Sodium chloride is an inorganic salt which is familiar as isotonizers for injections and eye drops. Sugar alcohols are polyhydric alcohols obtained by reducing carbonyl groups of sugar molecules and are also

2

familiar as diluents, isotonizers, stabilizers, etc. for oral preparations, injections and eye drops. Although sodium chloride and sugar alcohols are familiar as additives to pharmaceuticals, the purpose of addition in this invention is not conventionally simple isotonization but decrease of irritation which prevents GRF from being used as a nasal preparation. It is surprising that these generally used additives have such effects of decreasing irritation of a nasal preparation of GRF to nasal mucosa.

GRF is a peptide which shows growth hormone releasing activity and the activity is observed in several kinds of peptides each of which is composed of 44, 40, 37 or 29 amino acids. Their derivatives are also known. In this invention, any GRF is employed as long as it shows growth hormone releasing activity. For example, GRF may be any of the above GRF composed of 44, 40, 37 or 29 amino acids, their derivatives, analogues or a mixture thereof. GRF used in this invention may be obtained by extraction from living bodies, chemical synthesis or recombinant DNA method and it is not limited by the preparing method.

Nasal administration is a method to deliver medicines through nasal mucosa into the body and it is actually performed by spraying or dropping mainly aqueous solution into nasal cavity. Accordingly, preference is solution which may be sprayed or dropped. Lyophilized preparation may be prepared in consideration of stability of GRF in storage, which is used after reconstitution with a solvent at the time of use. Dosage form of this invention is not limited to those described in the above examples and may be others such as suspensions or gels.

Content of GRF in the preparations is usually 0.001 to 10 W/V%, preferably 0.01 to 10 W/V% in aqueous solution or suspension but this invention is not specifically limited by the content of GRF in the preparation.

Sugar alcohols used in this invention may be naturally obtained ones or artificially and chemically synthesized ones. Examples are mannitol or sorbitol.

The decreasing effect in irritation of a nasal preparation of GRF is obtained by simple adding sodium chloride and/or sugar alcohols to aqueous solution or suspension of GRF. These additives may be used singly or in combination of not less than two. For example, combinations of sodium chloride - mannitol, sodium chloride - sorbitol, sodium chloride - mannitol - sorbitol, and mannitol - sorbitol may be used. There is no limitation in an amount of these additives to GRF preparation in principle, but it may preferably be 0.01 to 10 W/V%, more preferably 0.1 to 3 W/V% in the case of sodium chloride and 0.1 to 30 W/V%, more preferably 1 to 15 W/V% in the case of sugar alcohols in an aqueous solution or suspension.

These additives may be added to either lyophilized preparation or reconstitution solvent when lyophilized dosage form is employed.

It is generally preferred in consideration of safety of GRF that osmotic pressure of a nasal preparation does not extremely differ from the physiological osmotic pressure and it is preferred in this invention that the relative osmotic pressure of the preparation is between 0.5 and 3.0 at the time of administration. The relative osmotic pressure described here is expressed by a relative ratio to the physiological osmotic pressure and that of 0.9 % aqueous sodium chloride solution is determined to be 1.

Preferred pH of a nasal preparation of GRF in this invention is 2 to 7 in consideration of stability of GRF.

The nasal preparation of GRF in this invention is prepared by a known method or a similar method thereto. For example, it is prepared by mixing, dissolving, dispersing, suspending, emulsifying or homogenizing necessary components at any order. Pharmaceutically acceptable additives such as buffers, stabilizers, isotonizers, solubilizers, disintegrators, dispersing agents, suspensioners, emulsifiers, preservatives and pH adjusters may be added, if necessary, to the preparation. However, these additives should not decrease solubility of GRF and should not give no good influence to processing of the GRF preparation at the targeted concentration. It is further preferred that a powdered formulation which is to be used after reconstitution at the time of use, is prepared by lyophilizing the obtained aqueous solution in consideration of stability of GRF in storage.

The present nasal preparation of GRF containing sodium chloride and/or sugar alcohols is low in irritation to nasal mucosa and gives no injury to nasal mucosa after a clinically effective amount of GRF is repeatedly administered to nasal cavity for a long period of time. Accordingly, the preparation of this invention is extremely suitable for nasal administration and it enables to administer GRF for a long period of time by a painless, simple and convenient method of patient's own spray or dropping to nasal cavity and to have growth hormone releasing activity effectively exhibited without adverse reactions.

The invention is explained in more detail and concretely by the following experiments and examples.

Experiment 1

In order to study the reducing effect in irritation of the nasal preparation of this invention, eye dropping tests were carried out to rabbits by a method described below and the irritation was compared with other GRF preparations.

A solution each of additives and GRF(1-44) in distilled water for injection was controlled to pH about 5 by adding an appropriate amount of hydrochloric acid or sodium hydroxide. These solutions were subjected to sterile filtration to obtain GRF preparations containing sodium chloride (sample 1), sugar alcohols (samples 2 and 3), other additives than the above (samples 4 and 5), respectively, and a low concentration GRF preparation containing an absorption enhancer (sample 6) and an aqueous solution of GRF without any additive (sample 7). Concentrations of additives and GRFs are shown in Table 1 where amounts of additives in samples 1 to 5 were controlled so that relative osmotic pressure of each aqueous solution would be about 1.

Table 1

| Sample | Additive (mg/ml) | | GRF (mg/ml) |
|---|---|---|---|
| 1 | Sodium chloride | (9) | 1 |
| 2 | D-Mannitol | (53) | 1 |
| 3 | D-Sorbitol | (53) | 1 |
| 4 | Glycine | (22) | 1 |
| 5 | Citric acid | (50) | 1 |
| 6 | Saponin | (10) | 0.5 |
| 7 | None | | 1 |

Fifty $\mu\ell$ each of the above experimental sample was dropped into eyes of 5 rabbits per group and irritation to eye mucosa was evaluated.

Evaluation was made by observation of eye condition after dropping; it was scored 0 when no blink was observed, 1 for 1 to 2 blinks, 2 for 3 to 5 blinks, 3 for not less than 6 blinks, and 4 when eyes were kept closed and not opened for not less than 5 seconds. The less score shows the less irritation to eye mucosa. Results are shown in Table 2. Obviously reduction of irritation was observed in the GRF preparations of this invention (samples 1 to 3). On the other hand, no reduction of irritation was observed by additives other than sodium chloride and sugar alcohols for simple isotonization (samples 4 and 5). The strongest irritation was observed in the sample 6 in which an absorption enhancer was added even though the concentration of GRF was lower than in other samples. Addition of the absorption enhancer enlarged irritation to mucosa.

As shown in the above results, reduction in irritation of GRF was specifically obtained by addition of sodium chloride or sugar alcohols.

Table 2

| Sample | | Evaluation | | | | | Total score |
|---|---|---|---|---|---|---|---|
| 1 | Rabbit No. | (1) | (2) | (3) | (4) | (5) | |
| | Score | 0 | 0 | 0 | 3 | 0 | 3 |
| 2 | Rabbit No. | (6) · | (7) | (8) | (9) | (10) | |
| | Score | 2 | 0 | 1 | 1 | 1 | 5 |
| 3 | Rabbit No. | (11) | (12) | (13) | (14) | (15) | |
| | Score | 2 | 2 | 1 | 1 | 1 | 7 |
| 4 | Rabbit No. | (16) | (17) | (18) | (19) | (20) | |
| | Score | 3 | 2 | 3 | 2 | 2 | 12 |
| 5 | Rabbit No. | (21) | (22) | (23) | (24) | (25) | |
| | Score | 2 | 2 | 2 | 3 | 3 | 12 |
| 6 | Rabbit No. | (26) | (27) | (28) | (29) | (30) | |
| | Score | 4 | 3 | 3 | 4 | 3 | 17 |
| 7 | Rabbit No. | (31) | (32) | (33) | (34) | (35) | |
| | Score | 3 | 3 | 3 | 3 | 2 | 14 |

Experiment 2

Experiment 1 was repeated except that GRF preparations containing sodium chloride (sample 8), sugar alcohols (samples 9 and 10), and sodium chloride and sugar alcohols (samples 11 to 13), respectively, GRF preparations containing other additives than sodium chloride and sugar alcohols (samples 14 and 15), a low concentration GRF preparation containing an absorption enhancer (sample 16) and an aqueous solution of GRF without any additive (sample 17). Concentrations of additives and GRF in each sample are shown in Table 3 where amounts of additives in samples 8, 9, 10, 14 and 15 were controlled so that relative osmotic pressure of each aqueous solution would be 1, and those in samples 11, 12 and 13 were controlled so that relative osmotic pressures would be 1.5 (= 1.0 based on sodium chloride plus 0.5 based on mannitol), 1.0 and 1.5 (= 1.0 based on mannitol plus 0.5 based on sodium chloride), respectively.

Table 3

| Sample | Additive (mg/ml) | | GRF (mg/ml) |
|---|---|---|---|
| 8 | Sodium chloride | (9) | 1 |
| 9 | D-Mannitol | (53) | 1 |
| 10 | D-Sorbitol | (53) | 1 |
| 11 | Sodium chloride<br>D-Mannitol | (9)<br>(26) | 1 |
| 12 | Sodium chloride<br>D-Mannitol | (4.5)<br>(26) | 1 |
| 13 | Sodium chloride<br>D-Mannitol | (4.5)<br>(53) | 1 |
| 14 | Glycine | (22) | 1 |
| 15 | Citric acid | (50) | 1 |
| 16 | Saponin | (10) | 0.5 |
| 17 | None | | 1 |

Results are shown in Table 4. Obviously reduction of irritation was observed in the GRF preparations of this invention (samples 8 to 13).

The irritation of aqueous solution of GRF without any additive (sample 17) is apparently reduced by addition of sodium chloride or sugar alcohols of this invention (samples 8 to 10). Furthermore, the same or more improved reduction in irritation was observed by addition of sodium chloride and sugar alcohols at the same time (samples 11 to 13), compared with the preparation containing sodium chloride or sugar alcohols.

On the other hand, no reduction of irritation was observed by additives other than those included in this invention (samples 14 and 15) and no reduction in irritation of GRF was observed by simple isotonization with materials other than sodium chloride or sugar alcohols. The strongest irritation was observed in the sample 16 in which an absorption enhancer was added even though the concentration of GRF was lower than that in other samples. Addition of the absorption enhancer enlarged irritation to mucosa.

As shown in the above results, reduction in irritation of GRF was specifically obtained by addition of sodium chloride or sugar alcohols and particularly, the reduction appeared very strongly in the GRF preparation containing sodium chloride and sugar alcohols at the same time.

Table 4

| Sample | | Evaluation | | | | | Total score |
|---|---|---|---|---|---|---|---|
| 8 | Rabbit No. | 211 | 216 | 218 | 222 | 223 | |
| | Score | 1 | 1 | 1 | 1 | 2 | 6 |
| 9 | Rabbit No. | 267 | 268 | 269 | 271 | 272 | |
| | Score | 1 | 2 | 2 | 0 | 2 | 7 |
| 10 | Rabbit No. | 330 | 331 | 332 | 333 | 334 | |
| | Score | 2 | 1 | 2 | 1 | 1 | 7 |
| 11 | Rabbit No. | 251 | 252 | 254 | 255 | 256 | |
| | Score | 1 | 1 | 2 | 1 | 1 | 6 |
| 12 | Rabbit No. | 257 | 258 | 259 | 260 | 261 | |
| | Score | 2 | 1 | 1 | 1 | 1 | 6 |
| 13 | Rabbit No. | 262 | 263 | 264 | 265 | 266 | |
| | Score | 2 | 1 | 1 | 0 | 0 | 4 |
| 14 | Rabbit No. | 311 | 312 | 313 | 314 | 316 | |
| | Score | 2 | 3 | 3 | 2 | 2 | 12 |
| 15 | Rabbit No. | 317 | 318 | 319 | 320 | 321 | |
| | Score | 3 | 2 | 2 | 3 | 3 | 13 |
| 16 | Rabbit No. | 322 | 323 | 324 | 325 | 326 | |
| | Score | 3 | 4 | 4 | 3 | 4 | 18 |
| 17 | Rabbit No. | 226 | 227 | 230 | 231 | 232 | |
| | Score | 3 | 3 | 3 | 3 | 3 | 15 |

Examples of this invention are presented below, but it is needless to say that this invention is not limited thereto.

## Example 1

In solution of sodium chloride (18 mg) in distilled water for injection (2 ml), was dissolved GRF(1-29) (2 mg) and pH of the resulting solution was adjusted to 5 by adding hydrochloric acid. This solution was subjected to sterile filtration to obtain a nasal preparation of GRF with low irritation. Relative osmotic pressure was about 1.

## Example 2

In solution of D-mannitol (30 mg) in distilled water for injection (1 ml), was dissolved GRF(1-44) (1 mg) and pH of the resulting solution was adjusted to 5 by adding hydrochloric acid. This solution was subjected to sterile filtration to obtain a nasal preparation of GRF with low irritation. Relative osmotic pressure was about 0.6.

## Example 3

7

In solution of sodium chloride (40 mg) in distilled water for injection (5 ml), was dissolved GRF(1-44) (10 mg) and pH of the resulting solution was adjusted to 5 by adding hydrochloric acid. The solution was subjected to sterile filtration and 1 ml each was dispensed in vials and lyophilized. After nitrogen gas was introduced, the vials were closed with rubber stoppers and cap-sealed to obtain nasal preparations of GRF with low irritation for reconstitution at the time of use. Relative osmotic pressure when reconstituted with 1 ml each of distilled water for injection at the time of use was about 0.9.

Example 4

In solution of D-sorbitol (150 mg) in glycine buffer (pH 5, 5 ml), was dissolved GRF(1-44) (15 mg). After the resulting solution was subjected to sterile filtration, 1 ml each was dispensed in vials and lyophilized. After nitrogen gas was introduced, the vials were closed with rubber stoppers and cap-sealed to obtain nasal preparations of GRF with low irritation for reconstitution at the time of use. Relative osmotic pressure when reconstituted with 1 ml each of distilled water for injection at the time of use was about 1.6.

Example 5

In solution of D-mannitol (250 mg) in phosphate buffer (pH 5, 10 ml), was dissolved GRF(1-29) (40 mg). After the resulting solution was subjected to sterile filtration, 1 ml each was dispensed in vials and lyophilized. After nitrogen gas was introduced, the vials were closed with rubber stoppers and cap-sealed to obtain nasal preparations of GRF with low irritation for reconstitution at the time of use each. Relative osmotic pressure when reconstituted with 1 ml each of distilled water for injection at the time of use was about 1.5.

Example 6

In solution of sodium chloride (68 mg) in distilled water for injection (5 ml), was dissolved GRF(1-44) (2.5 mg) and pH of the resulting solution was adjusted to 5 by adding hydrochloric acid. This solution was subjected to sterile filtration to obtain a nasal preparation of GRF with low irritation. Relative osmotic pressure was about 1.5.

Example 7

In solution of sodium chloride (270 mg) in distilled water for injection (10 ml), was dissolved GRF(1-29) (1 mg). After the resulting solution was subjected to sterile filtration, 1 ml each was dispensed in vials and lyophilized. After nitrogen gas was introduced, the vials were closed with rubber stoppers and cap-sealed to obtain nasal preparations of GRF with low irritation for reconstitution at the time of use. Relative osmotic pressure when reconstituted with 1 ml each of distilled water for injection at the time of use was about 3.

Example 8

In solution of D-mannitol (100 mg) and sodium chloride (18 mg) in distilled water for injection (2 ml), was dissolved GRF(1-44) (2 mg) and pH of the resulting solution was adjusted to 5 by adding hydrochloric acid. This solution was subjected to sterile filtration, to obtain a nasal preparation of GRF with low irritation. Relative osmotic pressure was about 1.9.

**Claims**

1. A low-irritative nasal preparation which comprises an effective amount of GRF and sodium chloride, or sugar alcohols or sodium chloride and sugar alcohols.
2. A low-irritative nasal preparation as claimed in Claim 1 characterised in that the said sugar alcohols are mannitol or sorbitol.

3. A nasal preparation as claimed in Claim 1 or Claim 2 characterised in that the GRF in the said preparation is present in an amount of 0.001 to 10 W/V%.

4. A nasal preparation as claimed in any one of Claims 1 to 3 characterised in that the GRF is a peptide composed of 44, 40, 37 or 29 amino acids, their derivatives, their analogues, or a mixture thereof.

5. A nasal preparation as claimed in any one of Claims 1 to 4 characterised in that the sodium chloride is present in an amount of 0.01 to 10 W/V%.

6. A nasal preparation as claimed in any one of Claims 1 to 5 characterised in that the sugar alcohols are present in an amount of 0.1 to 30 W/V%.

7. A nasal preparation as claimed in any one of Claims 1 to 6 characterised in that the pH of the said preparation is adjusted to 2 to 7.

8. A method of reducing irritation of nasal mucosa by adding sodium chloride, or sugar alcohols or sodium chloride and sugar alcohols to a GRF-containing nasal preparation.

9. A preparation as claimed in any one of Claims 1 to 7 for use as a medicament.

10. A nebulizer, or dropping bottle or resealable tube charged with GRF and sodium chloride NaC1 and/or one or more sugar alcohols.

11. The use of GRF and sodium chloride and/or one or more sugar alcohols in the preparation of a therapeutic composition for nasal administration having low or no irritative effect on the nasal mucosa.

European
Patent Office

**EUROPEAN SEARCH
REPORT**

Application Number

**EP 90 30 9377**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 301 392  (PIERREL SpA)<br>* Claims 1,2,6; examples 1-11 * | 1-6,8-11 | A 61 K 9/06<br>A 61 K 37/43 |
|  |  |  |  |
|  |  |  | **TECHNICAL FIELDS SEARCHED (Int. Cl.5)** |
|  |  |  | A 61 K |

The present search report has been drawn up for all claims

| Place of search | Date of completion of search | Examiner |
|---|---|---|
| The Hague | 18 December 90 | PEETERS J.C. |

CATEGORY OF CITED DOCUMENTS

X : particularly relevant if taken alone
Y : particularly relevant if combined with another
 document of the same catagory
A : technological background
O : non-written disclosure
P : intermediate document
T : theory or principle underlying the invention

E : earlier patent document, but published on, or after
 the filing date
D : document cited in the application
L : document cited for other reasons

&: member of the same patent family, corresponding
 document